# EUROPEAN PATENT SPECIFICATION

(11) **EP 3 627 514 B1**
(45) Date of publication and mention of the grant of the patent: **17.08.2022**
(21) Application number: 18020458.8
(22) Date of filing: 24.09.2018
(51) Int. Cl.: G16H 20/30, G16H 40/67, G16H 50/20

(54) **SYSTEM AND METHOD FOR OPTIMISED MONITORING OF JOINTS IN PHYSIOTHERAPY**
SYSTEM UND VERFAHREN ZUR OPTIMIERTEN ÜBERWACHUNG VON GELENKEN IN DER PHYSIOTHERAPIE
SYSTÈME ET PROCÉDÉ POUR OPTIMISER LA SURVEILLANCE DES ARTICULATIONS EN PHYSIOTHÉRAPIE

(30) Priority: 21.09.2018 RO 201800710
(43) Date of publication of application: 25.03.2020
(73) Proprietor: SC Kineto Tech Rehab SRL, 010917 Bucuresti (RO)
(72) Inventor: Kluger, Andrei, Bucuresti (RO); Moldoveanu, Camil, Bucuresti (RO)
(74) Representative: Stellbrink & Partner Patentanwälte mbB

(56) References cited:
- EP-A1- 3 064 134
- US-A1- 2017 143 261
- US-A1- 2017 265 800

## Description

### Technical Field

The invention presents a new system and the corresponding implementing method of monitoring patients in need of physiotherapy, both in the clinic and at home, by using three axes accelerometers and three axes gyroscopes as wireless motion sensors, attached around the injured joint, and detecting, monitoring and reporting the quality of the exercises patients are doing both to the physiotherapist and to the patient

It is based on a mobile application that receives sensor data and a web server where exercises and exercise programs are defined and scheduled for patients, by hosting a cloud-based database which keeps the physiotherapist, patient and exercise data.

Present invention is an optimized and customer oriented monitoring solution, by allowing both flexible placement of the motion sensors above and below the patient's affected joint, minimisation of the linear drift of the gyroscopes as well as an optimization of the exercise program based on big data analysis, derived from a gradual improvement of the program quality, learned from the patient's adherence and the previous responses of the physiotherapist

### Background Art___________

Background art reveals an important number of solutions built up around mobile applications and dedicated to systems and related methods for monitoring the process of rehabilitation by physiotherapy of the injured joints..

Practically all of them are using one or more devices for motion and/or position detection of the patient during the physiotherapy recovery process of the joints, a database containing relevant information on exercises, modules for comparing real execution with model exercise and making relevant assessments and means for particular calibration of the input information from sensors.

The solution as presented in (1), relates to a system and implementing method that proposes monitoring and correction of the execution of physical exercises by means of a Pilates type exercises device. Detection is ensured by a fixed Kinect motion sensor plus a video camera together with an infrared proximity sensor.

Accordingly, this solution is based on image processing and the calibration is intended not to detect position of the sensors around the joint but to adjust the position of the body and the exercises to the position of the sensors on the injured joint, which might create real and sensitive calibrating problems.

In (2), the described monitoring system and method is based on inertial sensors and a data processor for determining the movements of the patient and their correction against a predefined model. The implemented calibration procedure has the meaning of calibration using start and end positions of a specific movement and assumes these positions are fixed and known. If those positions are not achieved with accuracy by the patient, which in practice happens often, the errors of the calibration will be reflected in the subsequent angle measurement and will lead to incorrect measurements.

In document (3), the described monitoring system and method is practically based on an unspecified number and types of motion and position sensors: accelerometer, gyroscope, magnetometer, MEMS sensor, digital compass, inertial meter, temperature sensor, video camera, etc. Accordingly, the processor for sensor data is specific to the actually used sensors. An important limitation of this solution would be the lack of a specific module/algorithm for position calibration of the sensors.

Likewise, in (4), the number and types of sensors in the Sensors unit are not effectively specified, by leaving to the user the option of correlating with the software in the mobile application. The proposed calibration has the exclusive meaning of a selection of the rehabilitation programs and does not cover the usual calibration of the sensors' position.

In document (5), the entire solution of the monitoring system and related method, including the calibration unit is based on processing video information. Possible disadvantages of that solution reside in the impossibility of detecting the supine position, detection only for privileged angles (where the camera is not obstructed by the body) and diminished accuracy.

Patent document (6) describes a system where sensors are mounted on the patient's limbs in order to monitor the exercises done by the patient. The system uses sensors with a three axis accelerometer, three axis gyroscope and three axis compass.

Using a compass in a clinic environment means the sensor data is subjected to magnetic interferences from all the equipment available in the surroundings of the patient and data can be influenced and distorted.

The solution presented in said document requires an accurate calibration of the data from the sensors, which implies that one should know exactly the position of the sensors placed on the limb in order to measure accurately the angles between the segments of the limb.

In the disclosure of US 2017/0265800 A1, a motion sensor is attached to a patient's limb distal to a joint on which surgery has occurred and another motion sensor is attached to the limb proximate to the joint. The motion sensors may be accelerometers and gyroscopes that are capable of generating data that corresponds to flexion of the joint. Temperature sensors are attached to the patient at the surgical site at the joint and at a site separated from the surgical site so that the temperature difference between the sensors may be monitored. Data from the sensors is used to monitor the patient's post-surgical condition and rehabilitation and to provide feedback to the patient.

Some limitations appear to result from the cited background art, which indicates needs for improved or, more specific, optimised solutions.

The first one relates to sensitivity and accuracy of the data processed by the motion and position sensors used by respective systems, usually improved by calibration means.

Virtually all known systems, are based on a calibration procedure which assumes a very well determined position of the sensors around the injured joint, a process which could be inherently inaccurate, sometimes difficult and subject to local condition which could undermine the calibration process. If used by itself, the gyroscope has a linear drift which could contribute to an alteration of the information. The same is true for the accelerometers that have a high static noise. The compass is also very prone to magnetic errors when close to iron objects.

Besides problems derived from the cited patent literature, an overview of the background art reveals that progress on the flexion is now reported either by subjective means (depending on the physiotherapist's experience and knowledge) or by using a goniometer. The goniometer isn't very accurate and cannot be used all the time during the exercise. For an injured patient it's critical to see the evolution of the rehabilitation process, what he is doing wrong while performing the exercises, and this information is now lacking.

Most of solutions are using only a limited 2D representation and rendering of the injured joint and having the relevant angles displayed on a 3D representation on the mobile interface would allow the patient to have an objective information about his/her momentary progress and how he can strive to improve.

Recovery programs are not standardized; each physiotherapist is free to choose his own exercise programs, which means patients will not have the same quality of service across clinics or even the same clinic. There's only a very small amount of studies to determine which exercises work best for each type of affection. There are exercises that are prescribed for each type of affection but there's no additional data correlating the adherence and progress to the exercises and type of patient.

A very important progress indicator is the adherence of the patient to the treatment prescribed by the physiotherapist and this too isn't measured accurately, most of the time being subjectively checked by the physiotherapist or self reported by the patient (when doing the exercises at home).

There's great standardization in the surgeries performed by the doctors, but almost none in the recovery process, either recovery in the clinic or recovery at home. When measuring the success of the patient's recovery most of it is made from the physiotherapy which follows the surgery and only a small part depends on how well the surgery was done.

### Summary of invention__________________________

The technical problem raised and solved by this invention is minimizing and neutralizing the disturbing effect of the position of the sensors around the injured joint under physiotherapy monitoring, compensation of the gyroscope's dynamic drift and, based on the analysis of the received data, optimising the quality and timing of the prescribed exercises and thus ensuring the success in the patient's recovery.

The present invention is defined by the claims.

The solution of the present invention is built up within a mobile application and comprises a set of motion sensing devices mounted above and below the injured joint of a patient, a mobile application that receives sensor data and offers interfaces to the physiotherapist and the patient, relevant exercise definition, assessments and movement analysis, and respectively a web server which hosts a cloud-based database which keeps the physiotherapist, patient and exercise data. It allows the physiotherapist to prescribe a set of exercises,accurately monitor the patient's training and to objectively measure the progress of the patient in the clinic and at home which provides much more accurate info than the subjective one that might be offered by the patient.

Some important technical features have been considered by this invention in order to contribute in achieving an optimized physiotherapy monitoring of joints.

Firstly, the claimed invention calibrates the signals received from the motion sensors by a specific calibration algorithm which detects the actual positioning and ensures the orientations of the sensors is related to the actual physical axis of rotation of the joint, and thus the patient should not fix them in a certain position. This makes the solution very simple to use both for the physio in assessments and for the patients when performing the remote assisted exercises at home.

Secondly, the claimed invention ensures a dynamic gyro drift compensation which ensures the sensor data is immune to drift.

Another technical feature of the claimed invention is the implementation of a machine learning procedure meant to ensure a continuous assessment of the exercise program changes and program evaluation. The progress of the patient's flexion is automatically analyzed against patterns already existing in the database and depending on the detected improvements, it generates relevant notifications, alerts, and, finally, a better selection and scheduling of the exercises and automatic personalization of the patient schedule.

Having regard to similar solutions contained in the state of the art, one may conclude that this invention offers the following advantages:
- motion sensors are limited to accelerometer and gyroscope;
- by using calibration and compensation procedures, the solution is " flexible "to the placement of the said sensors anywhere above and below the affected joint, and compensates the dynamic drift of the gyroscope;
- solution ensures determination of the actual physical flexion axis of the joint with respect to the anatomical characteristics of the patient;
- solution enables detection and measurement of additional angles: abduction/adduction angle, hip flexion angle and hip rotation angle
- enables an automatic optimization of the exercise detection by using a machine learning procedure and thus , improves the patient's adherence by accurately identifying the "improved" movement the patient should do in order to recover more rapidly

### Brief description of drawings

A detailed description of the invention and its embodiments will be further presented with reference to the following figures:

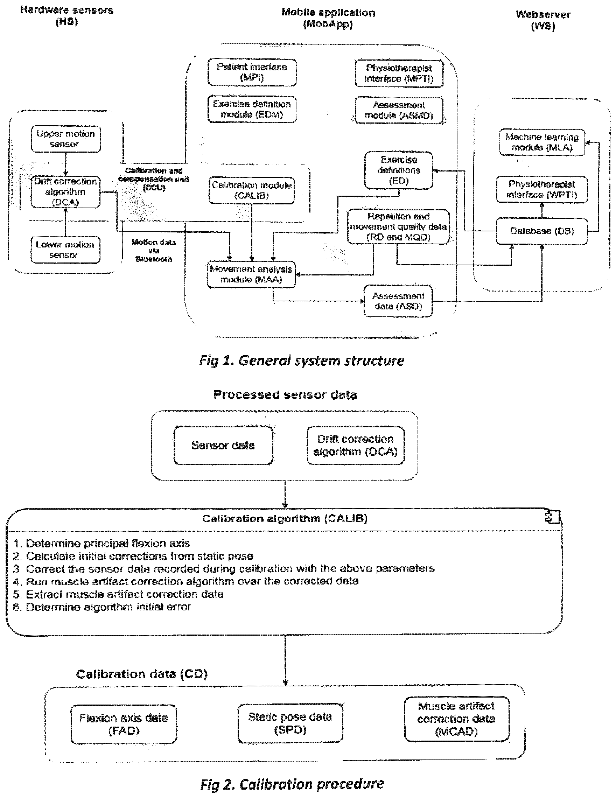

Figure 1- General system structure
Figure 2- Calibration and correction procedure

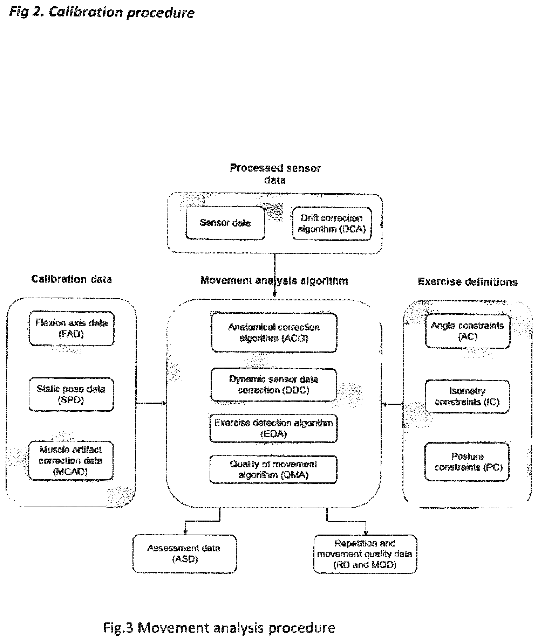

Figure 3- Movement analysis procedure

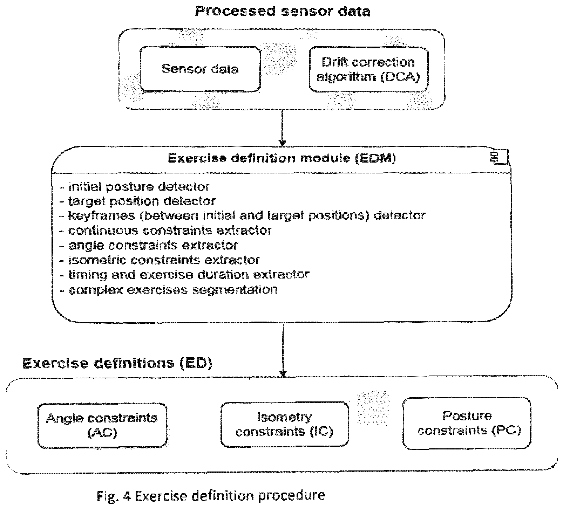

Figure 4- Exercise definition procedure

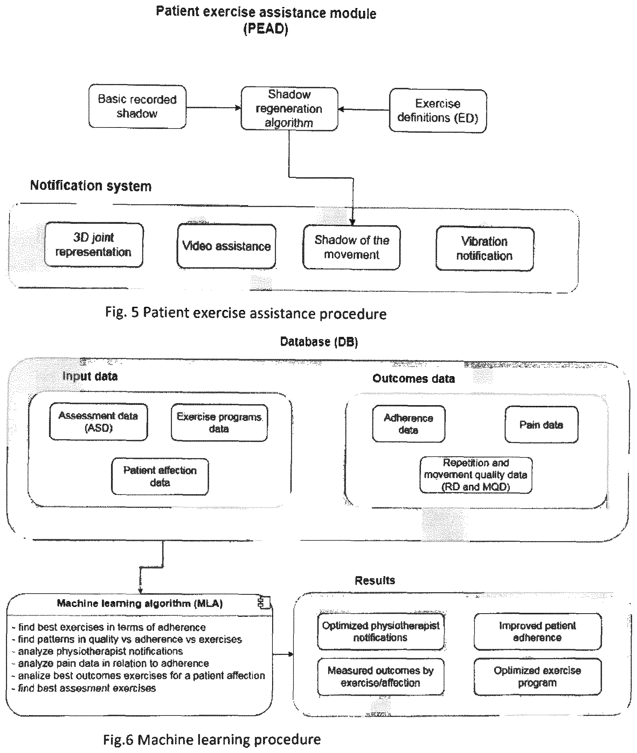

Figure 5 Patient exercise assistance procedures
Figure 6 Machine learning procedure

### Description of embodiments

The solution consists of a set of motion sensing devices HS mounted above and below the injured joint of a patient, a mobile (tablet or mobile device) application MobApp that receives sensor data, a web server WS where exercises and exercise programs are defined and scheduled for patients, a cloud-based database DB which keeps the physiotherapist, patient and exercise data.

The hardware sensors HS contain three axis accelerometers, three axis gyroscopes. Different from other motion tracking implementations, the system does not use a magnetometer as it's very susceptible to interference with iron based objects, which are very common in the physiotherapy clinics and even at home.

Data from the accelerometer and gyroscope is fused within the device to obtain a corrected position in space. This data offers the orientation in the absolute reference system of the sensor without it having a true north heading as the solution doesn't use the compass. By itself, the data offered by the each sensor is not relevant and it has to be processed by the mobile application in relation to the joint and the other sensor.

Using only gyroscopes and accelerometers without compass means that the orientation is prone to drift on the long term. A compensation procedure formalized within a related algorithm DCA compensates linear drift from the gyroscopes.

The placement of the sensors on the body is very important for other solutions as it influences all the subsequent measurements. This is why other solutions rely on very careful placement of the sensors that can be recognized by their algorithms. From our perspective it's very hard for the patient to accurately place the sensors in the desired position: the desired position might be over the injury, it's not comfortable to wear the sensors in those positions, the patient has different sized limbs than the standard, etc. Another issue is that careful placement means lost time and additional worries for the patient who should concentrate on recovering from the injury. This might affect the adherence to the treatment plan which is not desirable.

This is why the claimed solution allows the motion sensing devices to be placed randomly above and below the patient's affected joint. The patient doesn't have to fix them in a certain position; the calibration algorithm detects the actual positioning and ensures that the orientations of the sensors are related to the actual physical axis of rotation of the join.

The mobile application MobApp is made of two separate interfaces, a physiotherapists interface MPTI and a patient interface MPI. These two different interfaces are accessible using the corresponding login parameters, if the user logging in is a patient the patient interface is shown, if the user is a physiotherapist, the physiotherapist interface is shown instead.

The mobile application MobApp includes as well an Exercise definition module EDM, an Assessment module ASMD and a Movement analysis algorithm MAA.

The physiotherapist interface MPTI shows all the patients that physiotherapist has registered, their progress, the list of scheduled exercises, feedback from the patient and patient assessment module.

The patient assessment module ASMD allows the physiotherapist to objectively measure the phase the patient is in the recovery process, how well the injured joint behaves compared to the healthy one and, based on this, change the thresholds of exercises or even the exercise list, and compared also with an average individual, in the same age and activity group.

This assessment, and the data associated with it - ASD, is an extremely important part of the recovery process as in this phase the physiotherapist determines with the patient what is the expected outcome of the physiotherapy. This outcome can be functional recovery, professional athlete performance or anywhere in between. This outcome determines the length of exercise program, the intensity of the training, the intermediate goals and the actual exercises to be done.

By using the initial assessment and monitoring the patient all through the recovery process the patient is more motivated and has more feedback about how the recovery is progressing which leads to greater adherence to the program.

The physiotherapist has a database of exercises to choose from or predefined programs that can assign to a patient depending on the affection/surgery.

The exercise database contains a list of predefined exercises that the physiotherapist will use as a base for his own exercise programs. Each exercise can be customized within the physiotherapist application so it fits the patient using the system.

An exercise definition module allows the physiotherapist to record an exercise with the sensors on his body (or alternatively to instruct the patient to do an exercise with the sensors on) and then the exercise processing algorithm disseminates the data and sets all the constraints based on that recording. This allows the physiotherapist on one hand to easily customize the existing exercises and on the other hand enables him to create his own set of exercises if the ones in the platform are not enough.

The patient interface PMI contains a list of exercises prescribed by the physiotherapist to the patient using the web application. This list can be updated by the physiotherapist using the web platform or the mobile application. The mobile application receives data from the motion sensing devices, shows the movement live on the mobile device screen, counts exercise repetitions and shows how correct the movement was done using the thresholds the physiotherapist has previously set.

A calibration and compensation procedure formalized by the calibration algorithm CALIB and drift correction algorithm DCA is used on one side to ensure motion data is aligned with physical axis of movement and on the other side that the sensor data are drift immune. The algorithm uses a combination of a static pose and dynamic movements of the patients' joint to determine where the motion detection devices were placed on the patient's body and uses that information to correct the movement data accordingly.

The dynamic calibration movement is made around the main flexion axis of that joint i.e. for knee the motion is a knee flexion, for elbow it's an elbow flexion motion, for back it's a back bending motion, etc. This motion, repeated a number of times gives the raw principal axis of that joint FAD. Sensor positioning is inferred from this principal axis and then body position compensation is calculated. This uses previously determined muscle models, compensating for the movement of the sensors on the patient's body due to the muscle tissue and thus obtaining the muscle artifact correction data - MCAD.

A static pose, and the data that comes with it - SPD, is also taken during the calibration process to align the absolute coordinate systems of the two sensors. This static pose assumes that the joint of the patient should be straight and not flexed. If the patient cannot keep the joint straight as in the first days after surgery, the procedure allows for a correction angle inputted manually by the physiotherapist which is used to offset all the subsequent data from the sensors. This ensures the motion data is correct even for these specific cases where the patient is unable to fully extend the joint.

Further compensation is made taking into consideration the anatomical characteristics of the joint around which the sensors are placed. If the joint has certain degrees of freedom, the motion is checked and corrected against those parameters. For example, if it's a hinge joint there shouldn't be rotational movements in the recorded calibration motion.

Starting from the assumption that the gyroscope drift is linear with time, the double derivative of the angles returned by the gyroscope is calculated in the DCA algorithm, thus removing the linear part of the drift. To be able to reconstruct the angle a double integration is used which adds two sets of constants (one set for each integration operation). The first set is determined from the samples where the joint is not moving (in the static pose phase), for those samples it's equal to zero. The second one is determined as the initial orientation, and could be found in (7).

The drift is further eliminated when the two sensors readings are synchronized using the acceleration from the two sensors. This exploits the fact that the acceleration of one segment of the joint is the sum of the acceleration of the joint's center and the acceleration of that segment around the center. We apply this knowledge to determine the joint's center position in the local coordinates of each sensor allowing the angles to be determined with much reduced drift.

In order to implement the movement analysis procedure via the related movement analysis algorithm MAA, the relevant exercises are defined as a sequence of constraints that have to be met and an initial position that must be matched for the exercise to be started. The constraints are categorized by level of importance into layers, with layer 1 being the most important and layer 3 the least important. Each layer can have multiple constraints depending on the complexity or requirements of the exercise. Constraints can be defined as the target value, the tolerance of that value, when to check the respective value, if it's an isometric constraint and how much the patient needs to keep the body segments in that isometric position.

The time when the constraints are checked can be: on the peak of the movement, in the initial position, in the final position or continuous where the rule must apply to the whole exercise time.

The algorithm defines some standard initial postures from where the exercise starts, like standing, sitting, supine, side-lying, etc. but allows custom postures too. These custom postures are defined either manually by adding posture constraints PC or automatically where the physiotherapist uses the device to record a new exercise, by using the Exercise definition module - EDM and the algorithm analyses the collected data and generates an exercise definition ED complete with all the associated constraints AC, IC and PC.

Data from the motion detection devices is processed and fed through an Anatomical correction procedure and related algorithm, ACG, and a Dynamic sensor data correction module DDC. The ACG detect if any anatomical defined constraints were breached and assesses the breach and decides if the movement should be corrected accordingly. The DDC applies all the calibration data to the processed sensor data coming from the hardware sensors HS.

Both ACG and DDC are used to correct sensor placement adjustment, muscle movement and sensor decalibration during the time exercises are performed, and ensure the sensors keep their alignment to the meaningful physical axis at all times.

The movement data is then compared to the current exercise definition in an Exercise detection procedure and related algorithm EDA. If the exercise was done within the limits defined by the physiotherapist, a grade is generated in a Quality of movement algorithm QMA that represents the correctness of the exercise, taking into account the difficulty of the exercise and how well the constraints imposed by the physiotherapist were achieved. Visual, text and audio notification are sent to the patient to inform of the correctness of the exercise allowing the patient to improve the quality of the repetition.

An Exercise definition module EDM allows the physiotherapist to create a new exercise by putting sensors on the affected joint and doing the exercise he wants to add. The EDM extracts the definition of the exercise, in terms of constraints and lets the physiotherapist change it quickly.

The EDM records calibrated movement data, i.e. data that was corrected for sensor positioning on the body, and for muscle artifacts from the hardware sensors HS and then analyses that data in order to create the new exercise.

Initially, the initial posture recorded is compared to the standard postures already in the database. If there are major differences, a new custom posture is defined using posture constraints. These are expressed in angles and rotations that the application can measure. For example if it's a knee joint there can be hip angle, flexion angle, abduction angle, etc. If the sensors are placed on the back there can be lower back rotation, flexion, side inclination, upper back inclination, etc. The angles can be calculated from one segment to the other, or one segment to the body.

The target position is defined similar as the initial posture, using the angles that the application can measure.

If the exercise is a complex one and can be expressed as a succession of linear movement it is segmented into several parts and for each part a target and initial position is defined. This works similar to graphic animation where, from a sequence of key frames and a linear interpolation between them, an animation can be constructed.

Some of the angle constraints must be met during the whole exercise (for example keep the back straight while flexing it, keep the elbow straight while raising a weight with your hand or the knee straight while raising the foot in front of you). These are continuous constraints and the EDM suggests this too to the physiotherapist.

Some exercises count on keeping the joint in a certain position for an amount of time - these are called isometric exercises and are an important part of the recovery process. EDM also allows detection of isometric exercises and changing the time that should be spent in isometry.

EDM also analyses the data recording and extracts a timing for the exercises as some of them need to be done in a certain amount of time (either they need to be faster than an interval or they need to be slower than an interval).

An Assessment module ASD allows the physiotherapist to evaluate the patient at the start/end of important recovery phases and decide if the patient can go to the next phase or even finish the recovery process.

An assessment is made of a set of exercises specifically tailored to check the progress of the patient in certain areas that are important for the recovery process. The physiotherapist can measure objectively the end of a phase of recovery, how the patient performs at the end of the phase and how long it took to get there.

This is important in the recovery process as the patient does the same exercises as in the start of the phase and can compare the progress he's done in that phase.

For the physiotherapist it's a way of standardizing the outcomes of the recovery and measure individual milestones in the recovery process.

If the exercise is not done within the limits defined by the physiotherapist, the repetition is not counted and an audio, text and visual notification is sent to the patient to allow him to correctly perform the repetition for that particular exercise. This is done through a Patient exercise assistance module PEAD. This module is very important to the adherence and progress of the patient as it notifies the patient what he's doing wrong and when he is doing a wrong movement.

There are different types of assistance given to the patient performing the exercises.

The first type is the video assistance where a video of the exercise is shown, before the patient is expected to perform it. This is done to insure the patient remembers the exercise needed to be done.

The second type of visual assistance is the shadow of the movement: during the exercise a pre-generated shadow shows the patient how to perform the exercise. Having a standard shadow that would show how the joint of the patient should move would work only partially as each exercise is customized based on the progress of the recovery. For example, a patient immediately after a surgery can flex a joint only 50 degrees but after two weeks after the surgery he can flex for example to 90 degrees. This means the shadow has to be reconstructed based on the exercise constraints.

There is an algorithm built into the solution that allows a pre-recorded shadow of an exercise to be reconstructed when the constraints of that exercise are changed. The algorithm analyses the base shadow recording, segmenting it into keyframes like the start of the movement, the peak of the movement and the return of the movement. This allows the reconstruction of the shadow for most of the exercises. For movements that are not linear the algorithm analyses two differently defined shadows (for example one with target constraints at 30 degrees and one with target constraints at 80 degrees) and generates the movement curves corresponding to the two segments above and below the joint. By comparing those movement curves, the algorithm interpolates the positions and regenerates the shadow based on the new constraints.

For exercises that are complex and have a sequence of key frames defined, the shadow is regenerated individually for each segment taking into consideration all the exercise constraints and the individual segments timing.

The third type of visual assistance is a 3D real-time joint representation on which the sensors are fixed. The 3D rendition is moving on the screen of the mobile device synchronized with the actual physical joint and shows in real-time how the joint angles change during the movement. This allows the patient to see the angles while he's doing the movement and get feedback on what he's doing. The difference from other solutions that use a 2D render is that all the angles of the joint are being rendered, not only one of them, like in the 2D representation. By having the relevant angles displayed on the mobile interface the patient has objective information about his/her momentary progress and can strive to improve.

The last types of notifications are the vibration notification, where the hardware sensors (HS) vibrate when a movement is performed outside the bounds set by the physiotherapist, and respectively audio notification where a generated voice is telling the patient what is wrong with the movement he's just performed.

On the Webserver, WS, a machine learning procedure and its related algorithm MLA is implemented for exercise program changes and program evaluation.

The progress of the patient's flexion is automatically analyzed against patterns already existing in the database. If the algorithm detects the patient has progressed faster or slower than the exercise program he has scheduled, it generates an alert for the physiotherapist notifying the situation and asking for a change of program. If the patient has progressed faster, the physiotherapist is asked if the patient can be scheduled to complete exercises more advanced. If the patient has progressed slower, then the physiotherapist is asked if the current program should be repeated and the patient be notified.

The machine learning algorithm MLA learns from the responses that the physiotherapist sends and allows the system to show more relevant notifications.

An end result of the machine learning algorithm is to increase the adherence of the patients to the exercise program and detect which exercises work best for a category of patients and offer better outcomes than others for any patient.

Another important use of the algorithm is detecting patterns in patient exercise schedule and profile the patient into exercising categories. This allows for better scheduling of the exercises and automatic personalization of the patient schedule. For example, if the patient is profiled as one that will have low adherence over the program, then some exercises can be switched to some that may be easier to perform..

Data gathered from the sensors consist of, but is not limited to, joint position and orientation, physical movement angles values (flexion angle, hip, elbow, shoulder, etc angle, abduction angle, hip, elbow, shoulder, etc. rotation angle), acceleration, speed, time spent between repetitions and per set of exercises, maximum angle values during a repetition, correctness grade, detected initial and final posture.

The Web server acts also as a physiotherapist interface WPTI to the cloud-based database which stores all the exercise/programs data and the patient data. It allows defining new exercises by setting movement constraints and changing existing exercises. Continuous synchronization with the physiotherapist and patient mobile applications ensures the exercise database is always up to date.

The web platform allows exercises and exercise programs to be defined by the physiotherapist or by the clinic. There are standard public programs defined and available for all the clinics and physiotherapists, and private programs that can be seen only by the clinic.

The web platform allows the physiotherapist to see all the exercises done by the patient, the progress of the recovery and adjust the program scheduled for the patient.

The progress of the recovery can be measured in the first weeks of the recovery by the flexion increase. This is the phase where the patient tries to recover the lost flexion due to the injury. In this phase all progress and exercises are targeted towards the recovery of function for the patient. The phase is assumed to be completed when the patient recovers flexion to the values previous to the injury.

The next phase of recovery is targeted towards strengthening the muscles and recovering to the previous state before the injury. The progress in this phase is measured by the number of exercises done, the increased duration of isometric exercises and the increased weights used for the training.

### Citation List

### Patent literature

1. US 2012/0190505 A1- Method and system for monitoring and feed-backing on execution of physical exercise routines;
2. WO/2007/125344- Exercise monitoring system and method;
3. US 20170143261- System and methods for monitoring physical therapy and rehabilitation of joints;
4. US20170136296A1- System and method for physical rehabilitation and motion training;
5. US20080262772A1- System and a method for motion tracking using a calibration unit;
6. EP3096685A1- System and method for mapping moving body parts - Non Patent literature
7. R. Takeda, G. Lisco, T. Fujisawa, L. Gastaldi, H. Tohyama, S. Tadano-Drift Removal for Improving the Accuracy of Gait Parameters Using Wearable Sensor Systems; https://www.ncbi.nlm.nih.gov/pmc/articles/PMC4299060/

## Claims

1. A system for optimized physiotherapy monitoring of joints, comprising:
- a mobile application accessible by means of a mobile device;
- a web platform WS hosting a cloud-based database DB and a Machine learning unit MLU wherein said database DB contains a list of predefined exercises that the physiotherapist can use as a base for and exercise programs;
- a set of two motion sensing devices HS to be mounted above and below an injured joint of a patient, including at least a three axis accelerometer and a three axis gyroscope,
wherein the mobile application MobApp is configured to receive sensor data from the motion sensing devices HS anda comprises a physiotherapists interface MPTI and a patient interface MPI within a Patient exercise assistance module PEAD accessible using the corresponding login parameters, an Exercise definition module EDM, an assessment module ASD and a movement analysis module MAM,
wherein each exercise is defined as a sequence of constraints that have to be met and an initial position that must be matched for the exercise to be started,
wherein:
- said physiotherapists interface MPTI is accessible through the mobile device by using physiotherapist login parameters and is configured to visualize to the physiotherapist all the patients that physiotherapist has registered, their progress, the list of scheduled exercises, feedback from the patient and from said patient assessment module;
- said patient interface MPI is accessible through the mobile device by using patient login parameters and is configured to show to the patient:
∘ a list of exercises prescribed by the physiotherapist to the patient using the web platform and/or the physiotherapists interface MPTI of the mobile application,
∘ the movement live on the mobile device screen and
∘ how correctly the movement was done using thresholds the physiotherapist has previously set,
- said Patient exercise assistance module PEAD is configured to:
∘ provide a video assistance though said patient interface MPI by showing a video of the exercise before the patient is expected to perform it,
∘ pre-generate a shadow of the movement based on the exercise constraints and show it to the patient during the exercise;
∘ send to the patient, through said patient interface MPI, an audio, text and visual notification if the exercise is not done within the limits defined by the physiotherapist, to allow him to correctly perform that particular exercise;
- said Exercise definition module EDM is configured to create a new exercise by receiving sensor data from the motion sensing devices HS put on an affected joint while doing said new exercise;
- said assessment module ASD is configured to:
∘ compare the behavior of the injured joint compared to a healthy one, so allowing the physiotherapist to objectively measure the phase the patient is in the recovery process, how well and, based on this,
∘ allow the physiotherapist to change the thresholds of exercises or the exercise list;
- said movement analysis module MAM configured to check the constraints of each exercise, wherein the checking time of the constraint is either on the peak of the movement, in the initial position, in the final position or continuous, where the rule must apply to the whole exercise time,
wherein the web platform WS hosting the cloud-based database DB is configured to keep the physiotherapist, patient and exercise data,
wherein the mobile application includes a Calibration and compensation unit CCU, configured to:
- remove the linear part of the gyroscope drift by calculating the double derivative of the angles returned by the gyroscope;
- determine where the motion sensing devices were placed on the patient's body and correct the movement data accordingly using a combination of static pose data and dynamic movements data of the patients', wherein said determination includes:
∘ acquire said static pose data with the joint in a straight or offset from straight position to align the absolute coordinate systems of the sensing devices;
∘ acquire said dynamic movement data by means of a dynamic calibration movement made around a main flexion axis of the joint and repeated a number of times so as to obtain a raw principal axis of that joint.
∘ infer the motion sensing devices positioning from said raw principal axis;
- compensate a body position by using previously determined muscle models and compensating for the movement of the sensors on the patient's body due to the muscle tissue and thus obtaining a muscle artifact correction data.

2. A system according to claim 1 where the Machine learning unit MLU is arranged to learn from the responses of the physiotherapist and the quality of the exercises done by the patient, thus allowing the system to optimise the exercises program and identify relevant patterns in exercise schedule, improving relevant notifications, and profiling the patient into exercising categories and automatic personalization of the patient schedule.

3. A system according to claim 1 wherein the Calibration and compensation unit CCU is configured to:
- determine the principal flexion axis;
- calculate initial corrections from static pose;
- correct the sensor data recorded during calibration with the above parameters;
- run a muscle artifact correction algorithm over the corrected data;
- extract said muscle artifact correction data;
- determine algorithm initial error.

4. A system according to claim 1wherein wherein the Calibration and compensation unit CCU is configured:
- calculate the double derivative of the angles returned by the gyroscope;
- in order to reconstruct the angle, a double integration is used which adds two sets of constants, one set for each integration operation;
- determine the first set from the samples where the joint is not moving, in, the static pose phase, where for those samples it's equal to zero;
- determine the second one as the initial orientation;
- drift compensation when the readings of the two sensors are synchronized.

5. A system according to claim 1wherein the patient exercise assistance module is configured to:
- render the 3D image joint representation on the screen of the mobile device synchronised with the actual physical joint;
- show in real time how the joint angles change during the movement;
- allow the patient to see the angles while he's doing the movement and getting feedback on that.

6. A system according to claim 1 wherein the Machine Learning Unit is configured to:
- find best exercises in terms of adherence vs exercises;
- find patterns in quality vs. adherence exercises;
- analyze physiotherapist notifications;
- analyze pain data in relation to adherence;
- analyze best outcomes exercises for a patient affection;
- find best assessment exercises.

## Patentansprüche

1. System zur optimierten Physiotherapie-Überwachung von Gelenken, umfassend:
- eine mobile Anwendung, die mithilfe einer mobilen Vorrichtung erreichbar ist;
- eine Webplattform WS, die eine cloudbasierte Datenbank DB und eine Machine-Learning-Einheit MLU hostet, wobei die Datenbank DB eine Liste von vordefinierten Übungen enthält, die der Physiotherapeut als Grundlage für und Übungsprogramme verwenden kann;
- einen Satz von zwei Bewegungssensorvorrichtungen HS, die oberhalb und unterhalb eines verletzten Gelenks eines Patienten anzubringen sind und mindestens einen Drei-Achsen-Beschleunigungsmesser und ein Drei-Achsen-Gyroskop enthalten,
wobei die mobile Anwendung MobApp dazu eingerichtet ist, Sensordaten von den Bewegungssensorvorrichtungen HS zu empfangen, und eine Physiotherapeutenschnittstelle MPTI und eine Patientenschnittstelle MPI innerhalb eines Patientenübungsunterstützungsmoduls PEAD, auf das über die entsprechenden Anmeldeparameter zugegriffen werden kann, ein Übungsdefinitionsmodul EDM, ein Bewertungsmodul ASD und ein Bewegungsanalysemodul MAM umfasst,
wobei jede Übung definiert ist durch eine Sequenz von Bedingungen, die erfüllt werden müssen, und eine Ausgangsposition, die übereinstimmen muss, damit die Übung begonnen werden kann,
wobei:
- die Physiotherapeutenschnittstelle MPTI über die mobile Vorrichtung unter Verwendung von Physiotherapeuten-Anmeldeparametern erreichbar ist und dazu eingerichtet ist, dem Physiotherapeuten alle Patienten, die der Physiotherapeut registriert hat, ihren Fortschritt, die Liste der geplanten Übungen, Rückmeldungen des Patienten und von dem Patientenbewertungsmodul visualisiert;
- die Patientenschnittstelle MPI über die mobile Vorrichtung unter Verwendung von Patienten-Anmeldeparametern zugänglich und dazu eingerichtet ist, dem Patienten Folgendes anzuzeigen:
∘ eine Liste von Übungen, die der Physiotherapeut dem Patienten über die Webplattform und/oder die Physiotherapeuten-Schnittstelle MPTI der mobilen Anwendung verschrieben hat,
∘ die Bewegung live auf dem Bildschirm der mobilen Vorrichtung und
∘ wie korrekt die Bewegung ausgeführt wurde anhand der vom Physiotherapeuten vorab festgelegten Grenzwerte,
- das Patientenübungsunterstützungsmodul PEAD zu Folgendem eingerichtet ist:
∘ Bereitstellen einer Videounterstützung über die Patientenschnittstelle MPI, durch Zeigen eines Videos der Übung, bevor der Patient sie durchführen soll,
∘ Vor-Generieren eines Schattens der Bewegung auf der Grundlage der Bedingungen der Übung und Anzeigen desselben für den Patienten während der Übung;
∘ Senden einer akustischen, textlichen und visuellen Benachrichtigung an den Patienten über die Patientenschnittstelle MPI, wenn die Übung nicht innerhalb der vom Physiotherapeuten definierten Grenzen durchgeführt wird, um ihm die korrekte Durchführung dieser speziellen Übung zu ermöglichen;
- das Übungsdefinitionsmodul EDM dazu eingerichtet ist, eine neue Übung zu erstellen, indem es Sensordaten von den Bewegungssensorvorrichtungen HS empfängt, die auf ein betroffenes Gelenk gesetzt sind, während die neue Übung ausgeführt wird;
- das Bewertungsmodul ASD zu Folgendem eingerichtet ist:
∘ Vergleichen des Verhaltens des verletzten Gelenks im Vergleich zu einem gesunden Gelenk, so dass der Physiotherapeut objektiv die Phase des Genesungsprozesses, in der sich der Patient befindet, messen kann, wie gut er sich erholt hat, und basierend darauf,
∘ Ermöglichen, dass der Physiotherapeut die Grenzwerte von Übungen oder die Übungsliste ändert;
- das Bewegungsanalysemodul MAM dazu eingerichtet ist, die Bedingungen jeder Übung zu überprüfen, wobei der Zeitpunkt der Überprüfung der Bedingung entweder am Extrempunkt der Bewegung, in der Ausgangsposition, in der Endposition oder kontinuierlich ist, wobei die Regel für die gesamte Übungszeit gelten muss,
wobei die Webplattform WS, welche die cloudbasierte Datenbank DB hostet dazu eingerichtet ist, die Physiotherapeuten-, Patienten- und Übungsdaten zu speichern,
wobei die mobile Anwendung eine Kalibrierungs- und Kompensationseinheit CCU enthält, die zu Folgendem eingerichtet ist:
- Entfernen des linearen Anteils der Gyroskop-Drift durch Berechnung der doppelten Ableitung der vom Gyroskop gelieferten Winkel;
- Bestimmen, wo die Bewegungssensorvorrichtungen am Körper des Patienten platziert wurden, und entsprechendes Korrigieren der Bewegungsdaten unter Verwendung einer Kombination aus statischen Posedaten und dynamischen Bewegungsdaten des Patienten, wobei die Bestimmung Folgendes beinhaltet:
o Erfassen der statischen Posedaten, während sich das Gelenk in einer geraden oder von der geraden Position versetzten Position befindet, um die absoluten Koordinatensysteme der Sensorvorrichtungen auszurichten;
∘ Erfassen der dynamischen Bewegungsdaten mittels einer dynamischen Kalibrierungsbewegung, die um eine Hauptbeugeachse des Gelenks durchgeführt und mehrmals wiederholt wird, um eine grobe Hauptachse dieses Gelenks zu erhalten,
∘ Ableiten der Positionierung der Bewegungssensorvorrichtungen aus der groben Hauptachse;
- Kompensieren einer Körperposition, indem zuvor ermittelte Muskelmodelle verwendet werden, und Kompensieren der Bewegung der Sensoren am Körper des Patienten aufgrund des Muskelgewebes, um so Daten zur Korrektur von Muskelartefakten zu erhalten.

2. System nach Anspruch 1, wobei die Machine-Learning-Einheit MLU so eingerichtet ist, dass sie aus den Reaktionen des Physiotherapeuten und der Qualität der vom Patienten durchgeführten Übungen lernt, so dass das System das Übungsprogramm optimieren und relevante Muster im Übungsplan erkennen kann, wodurch relevante Benachrichtigungen verbessert werden und der Patient in Übungskategorien eingeordnet und der Patientenplan automatisch personalisiert wird.

3. System nach Anspruch 1, wobei die Kalibrierungs- und Kompensationseinheit CCU zu Folgendem eingerichtet ist:
- Bestimmen der Hauptbeugeachse;
- Berechnen von Anfangskorrekturen aus der statischen Pose;
- Korrigieren der während der Kalibrierung aufgezeichneten Sensordaten mit den oben genannten Parametern;
- Ausführen eines Algorithmus zur Korrektur von Muskelartefakten an den korrigierten Daten;
- Extrahieren der Muskelartefaktkorrekturdaten;
- Bestimmen des Anfangsfehlers des Algorithmus.

4. System nach Anspruch 1, wobei die Kalibrierungs- und Kompensationseinheit CCU zu Folgendem eingerichtet ist:
- Berechnen der zweiten Ableitung der vom Gyroskop gelieferten Winkel;
- um den Winkel zu rekonstruieren, wird eine doppelte Integration verwendet, bei der zwei Sätze von Konstanten hinzugefügt werden, ein Satz für jede Integrationsoperation;
- Bestimmen des ersten Satzes aus den Werte, bei denen sich das Gelenk nicht bewegt, in der Phase der statischen Pose, wobei für diese Proben der Wert gleich null ist;
- Bestimmen des zweiten Satzes als anfängliche Ausrichtung;
- Driftkompensation, wenn die Werte der beiden Sensoren synchronisiert sind.

5. System nach Anspruch 1, wobei das Patientenübungsunterstützungsmodul eingerichtet ist zu Folgendem:
- Wiedergeben der 3D-Bilddarstellung des Gelenks auf dem Bildschirm der mobilen Vorrichtung synchron mit dem tatsächlichen physischen Gelenk;
- Zeigen in Echtzeit, wie sich die Gelenkwinkel während der Bewegung verändern;
- Ermöglichen, dass der Patient die Winkel sieht, während er die Bewegung ausführt, und dass er Feedback dazu erhält.

6. System nach Anspruch 1, wobei die Machine-Learning-Einheit zu Folgendem konfiguriert ist:
- Finden der besten Übungen im Hinblick auf die Befolgung der Übungen;
- Finden von Mustern in Qualität vs. Befolgung der Übungen;
- Analysieren der Physiotherapeutenbenachrichtigungen;
- Analysieren von Schmerzdaten im Verhältnis zur Befolgung;
- Analysieren der Übungen mit den besten Ergebnissen für eine Patientenbehandlung;
- Finden der Übungen mit den besten Bewertungen.

## Revendications

1. Système de surveillance optimisée d'articulations en physiothérapie, comprenant :
- une application mobile accessible au moyen d'un dispositif mobile ;
- une plateforme Internet WS hébergeant une base de données basée sur le cloud DB et une unité d'apprentissage automatique MLU dans lequel ladite base de données DB contient une liste d'exercices prédéfinis que le physiothérapeute peut utiliser en tant que base pour des programmes d'exercices ;
- un ensemble de deux dispositifs de détection de mouvement HS à monter au-dessus et au-dessous d'une articulation blessée d'un patient, incluant au moins un accéléromètre à trois axes et un gyroscope à trois axes,
dans lequel l'application mobile MobApp est configurée pour recevoir des données de capteur des dispositifs de détection de mouvement HS et comprend une interface physiothérapeute MPTI et une interface patient MPI à l'intérieur d'un module d'assistance d'exercice patient PEAD accessible à l'aide des paramètres de connexion correspondants, un module de définition d'exercices EDM, un module d'évaluation ASD et un module d'analyse de mouvement MAM,
dans lequel chaque exercice est défini comme une séquence de contraintes qui doivent être satisfaites et une position initiale qui doit être adoptée pour que l'exercice commence,
dans lequel :
- ladite interface physiothérapeutes MPTI est accessible par l'intermédiaire du dispositif mobile à l'aide de paramètres de connexion physiothérapeute et est configurée pour que le physiothérapeute visualise tous les patients que le physiothérapeute a enregistrés, leurs progrès, la liste des exercices prévus, le retour du patient et dudit module d'évaluation patient ;
- ladite interface patient MPI est accessible par l'intermédiaire du dispositif mobile à l'aide de paramètres de connexion patient et est configurée pour montrer au patient :
° une liste d'exercices prescrits par le physiothérapeute au patient à l'aide de la plateforme Internet et/ou de l'interface physiothérapeute MPTI de l'application mobile,
° le mouvement en direct sur l'écran du dispositif mobile et
° le degré de correction avec lequel le mouvement a été exécuté à l'aide de seuils préalablement définis par le physiothérapeute,
- ledit module d'assistance d'exercices patient PEAD est configuré pour :
° fournir une assistance vidéo par l'intermédiaire de ladite interface patient MPI en montrant une vidéo de l'exercice avant que le patient ne soit supposé le réaliser,
° pré-générer une ombre du mouvement sur la base des contraintes d'exercice et la présenter au patient durant l'exercice ;
° envoyer au patient, par l'intermédiaire de ladite interface patient MPI, une notification audio, textuelle et visuelle si l'exercice n'est pas réalisé dans les limites définies par le physiothérapeute, pour lui permettre de réaliser correctement cet exercice particulier ;
- ledit module de définition d'exercice EDM est configuré pour créer un nouvel exercice par réception de données de capteur des dispositifs de détection de mouvement HS disposés sur une articulation affectée tout en réalisant le nouvel exercice ;
- ledit module d'évaluation ASD est configuré pour :
° comparer le comportement de l'articulation blessée à celui d'une articulation saine, permettant ainsi au physiothérapeute de mesurer objectivement la phase du processus de guérison à laquelle se trouve le patient, l'efficacité et, sur cette base,
° permettre au physiothérapeute de modifier les seuils d'exercices ou la liste d'exercices ;
- ledit module d'analyse de mouvement MAM configuré pour vérifier les contraintes de chaque exercice, dans lequel le temps de vérification de la contrainte est au sommet du mouvement, dans la position initiale, dans la position finale ou continu, lorsque la règle doit s'appliquer à toute la durée d'exercice,
dans lequel la plateforme Internet WS hébergeant la base de données basée sur le cloud DB est configurée pour conserver les données de physiothérapeute, de patient et d'exercice,
dans lequel l'application mobile inclut une unité d'étalonnage et de compensation CCU, configurée pour :
- retirer la partie linéaire de la dérive du gyroscope en calculant la double dérivée des angles renvoyés par le gyroscope ;
- déterminer où les dispositifs de détection de mouvements ont été placés sur le corps du patient et corriger les données de mouvement en conséquence en utilisant une combinaison de données de pose statique et de données de mouvements dynamiques des patients, dans lequel ladite détermination comprend :
° l'acquisition de données de pose statique avec l'articulation dans une position droite ou décalée d'une position droite pour aligner le système de coordonnées absolues des dispositifs de détection ;
° l'acquisition de données de mouvement dynamique au moyen d'un mouvement d'étalonnage dynamique réalisé autour d'un axe de flexion principal de l'articulation et répété un certain nombre de fois pour obtenir un axe principal brut de cette articulation ;
- la déduction de la position des dispositifs de détection de mouvement par rapport audit axe principal brut ;
- la compensation d'une position corporelle à l'aide de modèles musculaires préalablement déterminés et la compensation du mouvement des capteurs sur le corps du patient dû au tissu musculaire et ainsi l'obtention de données de correction d'artefact musculaire.

2. Système selon la revendication 1 où l'unité d'apprentissage automatique MLU est agencée pour apprendre des réponses du physiothérapeute et de la qualité des exercices réalisés par le patient, permettant ainsi au système d'optimiser le programme d'exercices et d'identifier des schémas pertinents dans le programme d'exercices, améliorant ainsi la pertinence des notifications, et définissant un profil du patient pour l'assigner dans des catégories d'exercices et la personnalisation automatique du programme patient.

3. Système selon la revendication 1 dans lequel l'unité d'étalonnage et de compensation CCU est configurée pour :
- déterminer l'axe de flexion principal ;
- calculer des corrections initiales à partir de la pose statique ;
- corriger les données de capteur enregistrées durant l'étalonnage avec les paramètres ci-dessus ;
- exécuter un algorithme de correction d'artefact musculaire sur les données corrigées ;
- extraire lesdites données de correction d'artefact musculaire ;
- déterminer une erreur initiale d'algorithme.

4. Système selon la revendication 1 dans lequel l'unité d'étalonnage et de compensation CCU est configurée pour :
- calculer la double dérivée et des angles renvoyés par le gyroscope ;
- de manière à reconstruire l'angle, une double intégration est utilisée qui ajoute deux jeux de constantes, un jeu pour chaque opération d'intégration ;
- déterminer le premier jeu à partir des échantillons où l'articulation est immobile, en phase de pose statique, où pour ces échantillons il est égal à 0 ;
- déterminer le second comme l'orientation initiale ;
- compenser une dérive lorsque les résultats des deux capteurs sont synchronisés.

5. Système selon la revendication 1 dans lequel le module d'assistance d'exercices patient est configuré pour :
- rendre la représentation de l'articulation en image 3D sur l'écran du dispositif mobile synchronisé avec l'articulation physique elle-même ;
- présenter en temps réel la manière dont les angles de l'articulation changent durant le mouvement ;
- permettre au patient de voir les angles alors qu'il réalise le mouvement et obtenir un retour sur cela.

6. Système selon la revendication 1 dans lequel l'unité d'apprentissage automatique est configurée pour :
- trouver les meilleurs exercices en termes d'adhésion vs exercices ;
- trouver des schémas d'exercices en termes de qualité vs adhésion ;
- analyser les notifications du physiothérapeute ;
- analyser les données de douleur relativement à l'adhésion ;
- analyser les exercices offrant les meilleurs résultats pour une affection d'un patient ;
- trouver les meilleurs exercices d'évaluation.
